# EUROPEAN PATENT APPLICATION

(11) **EP 4 811 418 A2**
(43) Date of publication of application: **23.09.2026**
(21) Application number: 26162287.2
(22) Date of filing: 04.03.2026
(51) Int. Cl.: H01J 49/00, G01N 27/623, G01N 33/68

(54) **FAST AND SELECTIVE MASS SPECTROMETRIC METHOD AND MASS SPECTROMETER SYSTEM FOR THE RELATIVE QUANTIFICATION OF ANALYTES**

(30) Priority: 19.03.2025 WO PCT/EP2025/057450
(71) Applicant: Roche Diagnostics GmbH, 68305 Mannheim (DE)
(72) Inventor: The designation of the inventor has not yet been filed
(74) Representative: Schach, Denise Kristine

(57) **Abstract**

Mass spectrometric method for determining a relative quantity of at least one analyte present in a first biological sample relative to the quantity of the same at least one analyte present in a second biological sample, the method comprising the steps of: providing the first biological sample comprising the at least one analyte labeled with a first isobaric mass tag; providing the second biological sample comprising the at least one analyte labeled with a second isobaric mass tag different from the first isobaric mass tag; mixing the first biological sample with the second biological sample to obtain a sample mixture; introducing, by direct inlet, a stream of the sample mixture into an ionization source; ionizing the stream of the sample mixture to generate labeled analyte ions of the at least one analyte labeled with the first and labeled with the second isobaric mass tag; separating the labeled analyte ions from other components of the sample mixture based on their ion mobility; fragmenting the separated labeled analyte ions to release reporter ions from the first and second isobaric mass tags; and measuring signal intensities of the reporter ions at their respective mass-to-charge ratios and determining therefrom the relative quantity of the at least one analyte present in the first biological sample relative to the second biological sample based on the measured signal intensities.

## Description

### Field of the Invention

The present invention relates to a mass spectrometric (MS) method and a mass spectrometer system for determining a relative quantity of at least one analyte present in a first biological sample relative to a second biological sample.

### Background of the Invention

In clinical research and In-vitro diagnostics (IVD), the precise quantification of proteins and peptides in complex biological matrices is essential for biomarker screening. While mass spectrometry (MS) is considered a gold standard for specificity and sensitivity in research settings, its widespread adoption into routine medical practice used to be rare and its impact as a reliable and highly selective technology for IVD has lately become more relevant.

Current standard workflows for peptide quantification typically rely on liquid chromatography which may be coupled to tandem mass spectrometry (LC-MS/MS), often utilizing nano- and/or micro-flow UHPLC systems combined with data-dependent acquisition (DDA) or data-independent acquisition (DIA). In these workflows, peptides are separated over a time gradient, which can typically range from 30 to 120 minutes, to reduce sample complexity before ionization. Quantification is then performed using methods such as label-free quantification (LFQ) or isotopic labeling (e.g., TMT, SILAC), all of which generally rely on the integration of chromatographic peak areas.

Known solutions based on LC-MS present several technical challenges that make them ill-suited for high-throughput environments like large clinical diagnostic laboratories. It is a time-consuming process where a single analysis requires substantial time for column equilibration, sample injection, gradient elution, and washing steps, restricting throughput to approximately 10-20 samples per day per instrument. This makes analyzing large clinical cohorts prohibitively slow and prevents the transition of proteomics from discovery research to routine clinical diagnostics.

Furthermore, the LC system itself is often the most fragile component of the analytical stack. Leaks, valve wear, and column clogging lead to frequent maintenance and significant instrument downtime. Accurate quantification in these systems often relies on reproducible retention times, but minor fluctuations in pump pressure or column aging can cause "drift," complicating data alignment across large sample batches. Additionally, carryover effects, where peptides from a previous run remain on the stationary phase and contaminate subsequent samples, pose a risk of false positives in diagnostic settings.

Moreover, the ionization instability caused by changing solvent compositions during gradient elution can affect ionization efficiency and signal stability dynamically.

### Summary of the Invention

It is therefore desirable to provide an improved method and system for analyte analysis, thereby increasing efficiency and/or throughput. Specifically, a method and a system are desirable that enable the quantification of proteins and peptides on a timescale of seconds to minutes per sample rather than hours, while maintaining high precision and robustness.

Further, it is desirable to provide a mass spectrometric method and system that eliminates run-to-run ionization variances and simplifies data processing by removing the need for retention time alignment. This approach may bridge the gap between high-depth proteomic research and the speed and robustness required for routine clinical diagnostics.

Moreover, it is desirable to provide a mass spectrometric method and system having elevated sensitivity and/or selectivity while at the same time achieving high efficiency.

At least one of these desired improvements can be achieved by the aspects of this disclosure, specifically the subject matter defined by the independent claims. Further advantageous effects and/or improvements being mentioned above or even exceeding such desired improvements are achieved by the specific embodiments, i.e. the corresponding subject matter covered by the dependent claims.

According to an aspect of the invention, which may be considered a first aspect, a mass spectrometric method for determining a relative quantity of at least one analyte present in a first biological sample relative to the quantity of the same at least one analyte present in a second biological sample, and optionally in a third, fourth, fifth, sixth, or a plurality of biological samples comprises the steps of: providing the first biological sample comprising the at least one analyte labeled with a first isobaric mass tag; providing the second biological sample comprising the at least one analyte labeled with a second isobaric mass tag different from the first isobaric mass tag; mixing the first biological sample with the second biological sample to obtain a sample mixture; introducing, by direct inlet, a stream of the sample mixture into an ionization source; ionizing the stream of the sample mixture to generate labeled analyte ions of the at least one analyte labeled with the first and labeled with the second isobaric mass tag; separating the labeled analyte ions from other components of the sample mixture based on their ion mobility; fragmenting the separated labeled analyte ions to release reporter ions from the first and second isobaric mass tags; and measuring signal intensities of the reporter ions at their respective mass-to-charge ratios and determining therefrom the relative quantity of the at least one analyte present in the first biological sample relative to the second biological sample based on the measured signal intensities.

The method according to the first aspect may provide a high degree of precision and throughput by eliminating run-to-run variances and significantly reducing the analysis time per sample. Further, the invention may provide a robust method and system that simplifies sample preparation and reduces the complexity of data processing compared to prior art liquid chromatography-mass spectrometry (LC-MS) workflows. Specifically, the invention relates to a workflow utilizing isobaric mass tagging which may enable high-throughput quantification in the field of clinical proteomics and quantitative biomarker screening.

The method according to the first aspect may provide the specific advantage of solving the problem of "ratio compression" often encountered in isobaric tagging experiments. By separating the labeled analyte ions based on their ion mobility prior to fragmentation, the method effectively acts as a gas-phase filter. This separation removes singly charged background ions, such as solvent clusters and/or lipids, and other isobaric interferences that would otherwise be co-isolated and fragmented into reporter ions, thereby distorting the quantitative ratios. Consequently, the accuracy of the relative quantification is significantly improved without the need for chromatographic separation.

Furthermore, the method may allow for a simplified data processing workflow. By introducing the sample mixture via direct inlet, without an upstream chromatography, the need for retention time alignment of chromatograms is eliminated. This removes a significant source of error related to chromatographic drift and reduces the computational resources required for data analysis. The simultaneous ionization of the analytes from the first and second biological samples ensures that they are subjected to identical ionization conditions, thereby physically eliminating run-to-run variances in ionization efficiency that typically affect sequential measurements.

A particular advantage of the method may at least for some embodiments be the targeted mitigation of ratio compression, a phenomenon typically caused by the co-isolation and co-fragmentation of interfering ions that share a similar mass-to-charge ratio with the precursor of interest. In traditional LC-free workflows, this problem is exacerbated by the high density of background ions.

At least some embodiments of the present invention address this by utilizing ion mobility as a high-speed gas-phase filter. By selecting specific collision cross-section (CCS) ranges, the method may selectively transmit multiply charged peptide ions while effectively filtering out singly charged background contaminants and chemical noise. This gas-phase purification may ensure that the resulting tandem mass spectra are dominated by reporter ions originating from the target analyte, thereby restoring the dynamic range of the quantification and ensuring that the measured ratios accurately reflect the biological concentrations in the original samples.

Unlike liquid chromatography-based systems, where the analyte is typically only available for measurement during a narrow temporal window as it elutes from the column, the direct inlet system of the present invention provides a continuous, steady-state stream of ions. This may enable 'sensitivity-on-demand', wherein the duty cycle of the mass spectrometer can be dynamically adjusted. If a specific analyte of interest may require higher sensitivity, the integration time for that specific ion mobility window or mass range can be extended without the risk of the 'peak' being missed or the chromatography gradient progressing. This decoupling of measurement time from separation time may allow for a highly flexible acquisition strategy where the depth of proteome coverage can be tuned in real-time based on the requirements of the specific clinical assay.

Specifically, at least some embodiments of the present invention depart from the long-standing paradigm in clinical proteomics that liquid chromatography (LC) is a mandatory requirement for the accurate quantification of complex proteomes. In the field, it is generally accepted that the omission of LC leads to prohibitive levels of ion suppression and spectral complexity, which would render the detection of low-abundance biomarkers impossible. However, according to some embodiments of the inventions, it has been surprisingly found that by combining a steady-state direct inlet with gas-phase ion mobility separation, the necessity for temporal separation is eliminated even in the case of multiplexing. This combination does not merely compensate for the lack of LC but actively exploits the continuous nature of the sample stream to achieve a level of physicochemical homogeneity in the ionized analyte population that is not possible with the transient peaks of traditional LC-MS. Consequently, the method may overcome the technical prejudice that high-throughput clinical screening requires a trade-off between speed and quantitative accuracy.

The term **"analyte"** and/or **"analyte of interest"** may refer to the chemical species to be analyzed via mass spectrometry. Chemical species suitable to be analyzed via mass spectrometry include but are not limited to amino acids, peptides, proteins (e.g. cell surface receptor, cytosolic protein etc.), metabolites, hormones, fatty acids, lipids, carbohydrates, and/or a substance that has been internalized by the organism (e.g. therapeutic drugs, drugs of abuse, toxins, etc.). In the specific context of isobaric mass tagging, the analyte may refer to a peptide and/or protein comprising a functional group, such as an amine group (e.g., N-terminus and/or lysine residue), capable of reacting with the labeling reagent.

The term **"biological sample"** (also denoted "sample") may in some cases refer to a part and/or piece of a tissue, and/or organ, typically being smaller than such tissue, and/or organ, intended to represent the whole of the tissue, and/or organ. Biological samples may include but are not limited to fluid samples such as blood, serum, plasma, synovial fluid, spinal fluid, urine, saliva, and lymphatic fluid, and/or solid samples such as dried blood spots and tissue extracts. Specifically, the first and second biological samples may respectively refer to a patient sample (e.g., from a subject to be diagnosed) and a control sample (e.g., a healthy control and/or a reference standard), or to two different samples from the same patient or from different patients. A control sample may hence refer to a healthy control and/or a reference standard (also denoted "internal standard"). A biological sample labeled with an isobaric mass tag different from the patient sample may function as an internal standard herein.

The term **"labeling reagent"** may refer to a chemical molecule used for quantitative mass spectrometry comprising of at least or consisting of three functional regions: a mass reporter, a mass normalizer (or balancer), and a reactive group. Labeling reagents are designed such that different labeling reagents in a set have the same total molecular weight (are isobaric) but contain a different distribution of heavy isotopes in the reporter and the normalizer regions. The mass reporter and mass normalizer of the labeling reagent may form a isobaric mass tag.

The term **"isobaric mass tag"** may refer to a chemical molecule and/or tag used for quantitative mass spectrometry comprising of at least or consisting of two functional regions: a mass reporter (or reporter ion) and a mass normalizer (or balancer). Isobaric mass tags are designed such that different tags in a set have the same total molecular weight (are isobaric) but contain a different distribution of heavy isotopes in the mass reporter and the normalizer regions. Consequently, analytes labeled with different tags in a set may be isobaric and/or co-migrated and/or co-existent in the mass spectrometer as a single precursor peak. Upon fragmentation (e.g., MS/MS), the bond between the mass reporter and the balancer cleaves thereby releasing **"reporter ions"** of distinct masses that can be individually resolved and quantified. Examples of isobaric mass tags include Tandem Mass Tags (TMT).

The term **"reporter ion"** may refer to a specific fragment ion released from an isobaric mass tag during fragmentation (e.g., collision-induced dissociation). The reporter ion may be indicative of the sample and/or the analyte of the sample. The signal intensity of the reporter ion may be directly proportional to the amount of the specific tag (and thus the analyte in the specific sample) present in the mixture. For example, in a TMTzero reagent, the mass reporter ion may be detected at a value of *m*/*z* 126.12.

The term **"direct inlet"** and/or **"direct infusion"** may specifically refer to a method of introducing a sample into the ionization source of a mass spectrometer without prior chromatographic separation of the components of the sample mixture over time and/or may refer to direct infusion without temporal chromatographic separation. "Direct inlet" may refer to the absence of temporal separation by chromatography during the analytical run itself. A bead-based enrichment step may however not be considered a (temporal) chromatographic separation. In a direct inlet approach, the stream of the sample mixture entering the ionizer may comprise the complex mixture of analytes simultaneously, rather than sequentially as in liquid chromatography. This approach is distinct from and devoid of a liquid chromatography column and/or a gas chromatography column upstream of the ionization source. This does however not exclude the possibility that chromatography is performed at some point in time before the biological samples are subjected to the method according to the first aspect or an embodiment described herein. In other words, the principle of direct inlet does not contradict the possibility that a sample was subjected to a chromatographic treatment before, for example before arriving in a lab where the method according to an embodiment is carried out. The user of the method may not always be in control of the treatments performed on a sample before it reaches the user. That may mean that the instrument(s) in the lab which carries/carry out the claimed method are completely free of a chromatography while the instruments preparing the sample out of the claimed scope and being separate from the instrument(s) in the lab which carries/carry out the method may have a chromatography. A user of the method may not always be able to tell whether a sample was subjected to chromatography.

The term **"ionization source"** and/or **"ionizer"** may refer to the device and/or interface within the mass spectrometer system that converts the neutral analyte molecules from the sample stream into gas-phase ions. Suitable ionization sources may include atmospheric pressure ionization sources, specifically electrospray ionization (ESI) and more specifically **"nano-electrospray ionization"** and/or **"nano-ESI".** Nano-ESI may refer to electrospray ionization operating at very low flow rates, typically in the range of about 200 nL/min to about 1000 nL/min, and/or even static nano-ESI using discrete emitters, which enhances ionization efficiency and sensitivity for small sample volumes.

The term **"ion mobility"** and/or **"separating based on ion mobility"** may refer to a gas-phase separation technique that distinguishes ions based on their size, shape, and charge state as they move through a buffer gas under the influence of an electric field. The parameter governing this separation is often the ion's collisional cross section (CCS). Specific implementations of ion mobility separation include, without limitation, Trapped Ion Mobility Spectrometry (TIMS), where ions are spatially accumulated and selectively released, Drift Tube Ion Mobility Spectrometry (DTIMS), Field Asymmetric Waveform Ion Mobility Spectrometry (FAIMS) and Traveling Wave Ion Mobility Spectrometry (TWIMS). Ion mobility may be used as a filter to separate the labeled analyte ions from background ions and isobaric interferences.

The term **"relative quantity"** may refer to the ratio of the amount and/or concentration of the analyte in the first biological sample compared to the amount and/or concentration of the same analyte in the second biological sample. It may be determined by calculating the ratio of the measured signal intensity of the reporter ion corresponding to the first isobaric mass tag to the measured signal intensity of the reporter ion corresponding to the second isobaric mass tag.

The mass spectrometric method for determining a relative quantity of at least one analyte present in a first biological sample relative to the quantity of the same at least one analyte present in a second biological sample may correspond to or may comprise an *in vitro* method.

The method may be configured for the simultaneous relative quantification of a plurality of patient samples against a common reference. In this embodiment, providing the biological samples may comprise providing a control sample (e.g., a pooled reference sample) labeled with a specific isobaric mass tag of a set of isobaric mass tags (e.g., the TMT-126 tag), and providing a plurality of distinct patient samples (e.g., 2-15 or more samples), each labeled with a unique, different isobaric mass tag from the same set (e.g., TMT-127 to TMT-134).

The step of mixing may comprise combining the labeled control sample with the plurality of labeled patient samples to form a multiplexed sample mixture. This multiplexed sample mixture may then be introduced into the ionization source via direct inlet as a single stream. Consequently, the analytes from the control sample and all patient samples may be ionized, spatially accumulated, separated by ion mobility, and fragmented, specifically simultaneously. The final mass-resolved spectrum may contain reporter ions for the control sample and each of the patient samples.

Determining the relative quantity may comprise calculating the ratio of the signal intensity of the reporter ion for each specific patient sample relative to the signal intensity of the reporter ion for the common control sample. This embodiment may advantageously allow, for example, for the high-throughput screening of a cohort of patients within a single acquisition cycle of the mass spectrometer (e.g., a single 30-second to 2-minute infusion), thereby drastically reducing the total analysis time per patient and ensuring that all patient results are normalized against the identical reference standard without run-to-run variations. Moreover, several samples of one single patient may be analyzed (against each other).

The step of providing of the first biological sample may be realized by and/or may correspond to providing a first reaction mixture that comprises: the first biological sample comprising the at least one analyte labeled with the first isobaric mass tag and at least one residual labeling reagent for labelling the at least one analyte with the first isobaric mass tag.

The step of providing of the second biological sample may be realized by and/or may correspond to providing a second reaction mixture that comprises: the second biological sample comprising the at least one analyte labeled with the second isobaric mass tag and at least one residual labeling reagent for labeling the at least one analyte with the second isobaric mass tag.

The step of providing the first biological sample comprising the at least one analyte labeled with a first isobaric mass tag, may, in one or more embodiments, comprise or correspond to mixing a first biological sample comprising the at least one analyte with a first labeling reagent.

In one or more embodiments after mixing the first biological sample comprising the at least one analyte with the first labeling reagent, the at least one analyte may react with the first labeling reagent to form the at least one analyte labeled with the first isobaric mass tag.

The step of providing the second biological sample comprising the at least one analyte labeled with a second isobaric mass tag, may, in one or more embodiments, comprise and/or correspond to mixing a second biological sample comprising the at least one analyte with a second labeling reagent.

In one or more embodiments after mixing the second biological sample comprising the at least one analyte with the second labeling reagent, the at least one analyte may react with the second labeling reagent to form the at least one analyte labeled with the second isobaric mass tag.

In one or more embodiments the at least one analyte may react in a nucleophilic substitution reaction with the first and/or second labeling reagent to form the at least one analyte labeled with the first and/or the second isobaric mass tag.

In one or more embodiments in the analyte labeled with the first isobaric mass tag, the first isobaric mass tag may be covalently bound to the analyte and/or in the analyte labeled with the second isobaric mass tag the second isobaric mass tag is covalently bound to the analyte.

In one or more embodiments the first labeling reagent and the second labeling reagent each may comprise a mass reporter, a mass normalizer (or balancer), and a reactive group, wherein the reactive group is an amine-reactive group.

The term **"amine-reactive group"** may refer to a functional chemical group that is configured to react with amines, preferably primary amines.

In one or more embodiments the amine-reactive group may comprise an N-hydroxysuccinimide (NHS) ester. Such an NHS-ester is preferably configured to efficiently and specifically form a covalent bond with primary amines, such as for example those present at the N-terminus of peptides or proteins and/or at the epsilon-amino group of lysine residues, preferably those present at the N-terminus of peptides or proteins.

In one or more embodiments the analyte may correspond to or may comprise a peptide and/or protein comprising a primary amine group, more preferably a primary amine group present at the N-terminus of the peptide or protein and/or at the epsilon-amino group of lysine residues. Most preferably the peptide and/or protein comprises only one primary amine group. The analyte may for example be Leucin-Enkephalin.

In further embodiments, the mass spectrometric method may further comprise providing a third, fourth, fifth, sixth, and/or a plurality of further biological samples, each comprising the at least one analyte. The at least one analyte in each of these additional biological samples is labeled with a corresponding third, fourth, fifth, sixth, and/or further isobaric mass tag, respectively, such that each isobaric mass tag is different from the first, the second, and/or any other isobaric mass tag used within the sample mixture.

It is to be explicitly understood that all features, characteristics, definitions, and method steps described herein with reference to the first and second biological samples and their respective first and second isobaric mass tags may apply equally and mutatis mutandis to the third, fourth, fifth, sixth, and any further biological samples and their corresponding isobaric mass tags. This includes, but is not limited to, the provision of the respective samples by mixing them with a corresponding labeling reagent, the structure of the labeling reagents (comprising a reactive group, a mass normalizer, and a mass reporter), the nucleophilic substitution reaction, the resulting covalent bonds, and/or the fact that all utilized isobaric mass tags of a set share the same total molecular weight while yielding reporter ions of different molecular weights upon fragmentation. This consequently enables the simultaneous relative quantification of the analyte across three, four, five, six, or even more biological samples in a single multiplexed spectral scan.

The step of mixing the first biological sample with the second biological sample to obtain a sample mixture may correspond to mixing the first reaction mixture with the second reaction mixture. This mixing step may in some cases be followed by diluting the sample mixture in an acidified organic solvent.

Mixing the reaction mixtures which contain residual labeling reagents and their subsequent diluting in an acidified organic solvent may be beneficial when bypassing liquid chromatography (LC) and/or solid-phase extraction (SPE). While conventional workflows (e.g., TMT-LC-MS) often subject reaction mixtures to SPE or C18 "spin tips" to remove salts (e.g., TEAB buffer) and/or unreacted labeling reagents before analysis, this purification can bear the risk of a loss of sample material, particularly for low-abundance analytes and/or very small sample volumes. By substituting the physical purification step (i.e. substituting chromatography) and using in addition a chemical stabilization via specific dilution in an acidified organic solvent, these embodiments help to avoid the need for such chromatographic cleanup. This approach may retain a high amount (e.g., potentially up to about 100%) of the analyte content available after the labeling reaction, thereby facilitating increased overall sensitivity for low-input samples (e.g., biopsies, single-cell proteomics) and reducing the variability introduced by manual or automated purification columns.

Furthermore, at least some of these features may support the stable ionization of complex matrices directly, which is beneficial for a robust chromatography-free direct inlet. While directly infusing a reaction mixture containing high salt concentrations (from buffering agents like TEAB) and unreacted tags could lead to unstable electrospray, nozzle clogging, or ion suppression, the specific acidification and organic dilution described herein may help to stabilize the nano-electrospray, assisting in neutralizing these matrix effects without necessarily requiring chromatographic separation.

The acidified organic solvent may comprise at least one of: acetonitrile (ACN); methanol (MeOH); ethanol (EtOH), specifically the acidified organic solvent may comprise: about 60-98 vol% ACN and about 0.02-0.4 vol% FA with the balance to 100 vol% being water, specifically about 70-95 vol% ACN and about 0.05-0.15 vol% FA, with the balance to 100 vol% being water and more specifically about 85-93 vol% ACN and about 0.08-0.12 vol% FA, with the balance to 100 vol% being water. For example, the acidified organic solvent may comprise or consist of about 90 vol% ACN, about 0.1 vol% FA and about 9.9 vol% water.

The specific dilution in an acidified organic solvent (e.g., high-percentage Acetonitrile with Formic Acid) may serve multiple physicochemical functions simultaneously:
It may maintain the solubility of the hydrophobic isobaric tags and the labeled peptides while preventing the precipitation of salts that could clog the nano-emitter.

The high organic content may lower the surface tension of the droplet, facilitating the formation of the Taylor cone and stable nebulization in the static nano-ESI source without the need for chromatographic focusing.

The acid component may enhance and/or support the complete protonation of the labeled peptides, which is important for efficient ionization in positive mode and optimal detection by the mass spectrometer.

At least some of these features may enable a stable "continuous spray" from a "dirty" matrix for the duration required (e.g., >30 seconds) to accumulate sufficient signal, which was previously considered very difficult or even unfeasible for such complex mixtures without clean-up, specifically without chromatography performed upstream the ionization.

Importantly, the workflow efficiency can be significantly increased having a direct impact on the increased throughput. The time-consuming steps of evaporation, reconstitution, and column equilibration associated with SPE clean-up can be avoided and/or bypassed. The sample is passed from "reaction vial" to "ion source" with only a simple pipetting/dilution step. This may drastically reduce the sample preparation time and/or cost per sample, enabling the high-throughput processing rates required for clinical diagnostics.

The use of a sample mixture with a high organic content, particularly an acetonitrile concentration exceeding 60% and preferably reaching approximately 90%, may provide a synergistic effect with the direct inlet nano-electrospray ionization. In the absence of a chromatographic gradient, this high organic load may ensure high spray stability and may improve the desolvation efficiency of the labeled peptides. This specific solvent environment may contribute to the reduction of cluster formation and may promote the formation of multiply charged species, which are subsequently more effectively separated from the matrix background by the ion mobility filter. This chemical optimization of the ionization environment may be an important factor in achieving the required limits of detection for clinical analytes in an HPLC-free workflow.

The term **"reaction mixture"** may refer to the crude solution resulting immediately from the chemical labeling process. It may comprise the biological sample (analytes), the reaction buffer (e.g., Triethylammonium bicarbonate (TEAB), HEPES), the isobaric labeling reagent (e.g., TMT), and in some embodiments the reaction byproducts and/or quenching agents (e.g., hydroxylamine). It may comprise "residual labeling reagents", i.e. unreacted isobaric tags that have not bonded to an analyte. Unseparated eluate may refer to the liquid sample mixture as it exists after labeling and dilution, containing analytes, residual reagents, and matrix components that have not been subjected to temporal separation via a stationary phase.

The term **"residual labeling reagents"** may refer to the excess labeling reagents (e.g., TMT reagents) remaining in the solution after the labeling reaction has reached equilibrium and/or has been quenched. In prior art methods, these are often considered contaminants to be removed; in the present embodiment(s), they may be tolerated within the mixture introduced into the ionizer without compromising the high degree in sensitivity and selectivity. The presence of these residuals may be a characterizing feature of a sample mixture when bypassing clean-up steps.

The term **"acidified organic solvent"** may refer to a solution comprising a major proportion of an organic solvent and a minor proportion of an acid. Specifically, the acidified organic solvent may be configured to provide a physicochemical environment that maintains analyte solubility while simultaneously lowering surface tension to facilitate Taylor cone formation in a chromatography-free nanoESI source. While specific concentrations such as 90% ACN and 0.1% FA may be preferred, the invention encompasses any concentration of organic solvent and acid that achieves stable steady-state ionization of a crude reaction mixture

The **"organic solvent"** may be selected to be compatible with electrospray ionization and capable of dissolving the labeled analytes. Preferred examples may include acetonitrile (ACN), methanol (MeOH), isopropanol (IPA), and/or mixtures thereof.

The **"acid"** may comprise a volatile acid suitable for mass spectrometry, configured to provide protons for ionization. Preferred examples may include formic acid (FA), acetic acid, and/or trifluoroacetic acid (TFA).

The step of separating the labeled analyte ions based on their ion mobility may comprise separating the labeled analyte ions from singly charged background ions and/or isobaric interferences.

The ion mobility separation step may act as a high-speed gas-phase filter mechanism that is particularly useful in chromatography-free workflows. Specifically, by distinguishing ions not just by mass, but by their spatial configuration, i.e. the Collisional Cross Section (CCS), the method may effectively de-convolute the 'precursor ion population' from 'isobaric interferences' and 'singly charged background ions'. This may be particularly advantageous for high-throughput clinical samples where chromatographic cleanup is bypassed; the ion mobility separator may compensate for the lack of temporal separation by preventing the co-fragmentation of off-target ions, thereby eliminating the 'ratio compression' effect and ensuring that reporter ion intensities remain proportional to the actual analyte concentration in the biological samples.

More specifically, in the absence of liquid chromatography (LC), the ionization source may simultaneously introduce a high-density population of analytes, solvents, and matrix-derived contaminants into a mass spectrometer. By utilizing an ion mobility separator-such as a Trapped Ion Mobility Spectrometry (TIMS) device-ions are distinguished not merely by their mass-to-charge (*m*/*z*) ratio, but by their CCS and charge state. This may allow a system to physically divert singly charged background ions (e.g., solvent clusters and lipids) and isobaric interferences (off-target ions with near-identical *m*/*z*) away from the target analyte path. The primary technical advantage of this 'gas-phase purification' may be the elimination of ratio compression. In traditional workflows, the co-fragmentation of these background interferences along with the TMT-labeled precursor might likely contaminate the reporter ion signal, artificially skewing the measured ratios toward unity. By ensuring that only the purified, multi-charged labeled analyte ions reach the fragmenter, the method may maintain high quantitative accuracy and dynamic range even when analyzing complex 'dirty' matrices directly from the reaction mixture. Furthermore, this separation may occur on a millisecond timescale, effectively recovering the analytical peak capacity lost by bypassing LC while maintaining the high-throughput benefits of direct infusion

The term **"Singly charged background ions"** may refer to and/or comprise chemical noise ubiquitous in complex biological matrices, including but not limited to solvent clusters, plasticizers, and/or lipids. Unlike the analytes of interest (typically peptides), which often carry multiple charges (z > 2), these background ions may be predominantly singly charged (z = 1).

The term **"Isobaric interferences"** may comprise "co-isolated" ions that possess a nearly identical mass-to-charge (*m*/*z*) ratio to the labeled precursor ion. In a traditional setup, these might be fragmented alongside the analyte, contributing "impurity" reporter ions negatively impacting the results.

The term **"ratio compression"** may refer to the phenomenon in isobaric labeling experiments where the measured signal intensities of reporter ions are skewed toward a 1:1 ratio (unity) due to the co-isolation and co-fragmentation of interfering ions. This effect typically masks the true biological differences in analyte concentration between samples. Ion mobility separation may act as a gas-phase filter to remove these interferences, thereby mitigating or eliminating ratio compression

The step of ionizing the stream of the sample mixture may be at least partially performed by means of a static nano-electrospray ionization (nanoESI) providing a continuous spray of the sample mixture.

In the specific embodiments utilizing static nanoESI, the method may enable the generation of a steady-state signal for the labeled analyte ions. This allows for the digital accumulation of mass-resolved spectra over a flexible measurement time period, decoupling the achievable sensitivity from the transient nature of a chromatographic peak. Thus, the signal-to-noise ratio can be enhanced by extending the acquisition time until a pre-defined statistical confidence threshold is reached.

In other words, the use of static nano-electrospray ionization (nanoESI) providing a continuous spray is advantageous when replacing the functionality of liquid chromatography. In traditional LC-MS, it is typically required to capture data within the narrow 'transient' window of an eluting peak. By utilizing a continuous spray from a static nano-emitter, the present embodiment transitions to a steady-state acquisition mode. This allows for the digital accumulation of signal over an extended duration, effectively using measurement time as a variable to increase sensitivity and statistical confidence. Furthermore, the low-flow characteristics of the nanoESI source significantly enhance ionization efficiency and matrix tolerance, allowing the system to stably ionize crude reaction mixtures containing residual labeling reagents and salts without the risk of emitter clogging or significant ion suppression typically associated with direct infusion of unpurified biological samples

The term **"static nano-electrospray ionization"** (Static nanoESI) may refer to an ionization process where a discrete, low-volume sample is loaded into a metallized glass capillary (emitter), and ionization is initiated by applying a voltage without an active mechanical pump or flow-rate gradient.

The term **"continuous spray"** may, unlike the transient, time-limited peaks produced during liquid chromatography (LC) elution, refer to a continuous spray which provides a steady-state signal of the sample mixture for an extended, flexible duration.

The term **"steady-state signal"** may refer to a continuous ion signal generated by the ionization source that remains substantially constant over a flexible measurement duration. This is distinct from the "transient" or time-limited signal peaks (which are conventionally produced during liquid chromatography elution, and which are restricted to a narrow temporal window). A steady-state signal allows for the digital accumulation of spectra over an extended period to increase sensitivity and statistical confidence.

The stream of the sample mixture may be provided as a constant stream, specifically the constant stream may be provided for a duration of at least about 10 seconds, specifically of at least about 20 seconds and more specifically of at least about 30 seconds.

Alternatively or in addition, the step of measuring signal intensities may comprise recording a plurality of mass-resolved spectra over a measurement time period while the stream is introduced, and accumulating said spectra to obtain a final mass-resolved spectrum, specifically the measurement time period may be between about 5 seconds and 30 minutes, specifically between about 10 second and 20 minutes and more specifically between about 15 seconds and 10 minutes.

The provision of the sample mixture as a constant stream for a duration of at least 30 seconds has a strong impact on the acquisition time and data quality. In traditional workflows, sensitivity is limited by the transient nature of a chromatographic peak. By utilizing a steady-state stream, the present embodiment may enable digital accumulation of spectra over a flexible measurement time period, which may range from about 15 seconds up to about 10 minutes depending on the required analytical depth. This approach may allow for online monitoring of data quality; the measurement may be concluded, in some embodiments, only when a pre-defined statistical threshold, such as a Coefficient of Variance (CV) of about 20%, is reached. This 'sensitivity-on-demand' capability may ensure that the relative quantification is robust against varying sample concentrations and physically eliminates the computational burden of retention time alignment and chromatographic peak integration.

This approach may allow for online monitoring of data quality; the measurement may be concluded, in some embodiments, only when a pre-defined statistical threshold, such as a Coefficient of Variance (CV) of about 20%, is reached. Depending on the complexity of the matrix and the abundance of the target analytes, this accumulation may occur over a duration ranging from several seconds to 30 minutes or more, thereby ensuring analytical depth is maintained even in the absence of chromatographic focusing.

The mass spectrometric method may comprise a simultaneous analysis of the first and second biological samples in a single spectral scan. In some embodiments, the mass spectrometric method may comprise a simultaneous analysis of the first and the second biological samples, together with a third or even more biological samples in a single spectral scan. For example, the first and second biological samples may respectively refer to a patient sample (e.g., from a subject to be diagnosed) and a control sample (e.g., a healthy control and/or a reference standard), or to two different samples from the same patient or from different patients. If more than two biological samples are analyzed simultaneously in a single spectral scan, such as three, four, five, six or even more, such as up to twenty or more, the multiplexing potential of such embodiments is exploited and multiple patient samples (of one single patient and/or several patients) may be analyzed at the same time, saving resources and time being often crucial in cases where a diagnostic result is urgently required.

The simultaneous analysis of the first and second biological samples, or even more biological samples in a single spectral scan provides a fundamental physical advantage by ensuring that all analytes are subjected to identical physico-chemical conditions throughout the entire analytical workflow. Unlike traditional sequential liquid chromatography-mass spectrometry (LC-MS) methods that rely on separate runs for each sample, the present method ionizes the labeled analytes from the mixed sample mixture at exactly the same time. This approach physically eliminates run-to-run ionization variances and signal instability caused by fluctuating source conditions or solvent compositions. Furthermore, because the first and second samples (and potentially a plurality of patient samples against a common reference) are processed as a single, co-existing ion population, the need for complex and error-prone retention time alignment of chromatograms may be entirely removed. This simultaneous acquisition may enable high throughput multiplexing where relative quantification ratios are determined with superior precision, as any fluctuations in the ionization source affect the reporter ions of both the first and second samples proportionally within the same spectral acquisition cycle.

The sample mixture may comprise a plurality of at least three (preferably at least five, more preferably at least ten) different biological samples, each labeled with a different isobaric mass tag, and wherein the introduction of the stream of the sample mixture may provide a steady-state signal of the labeled analyte ions during the steps of separating, fragmenting, and measuring, such that the relative quantities of the at least one analyte across all samples in the plurality are determined simultaneously from the same steady-state ion population.

While in LC-MS, "multiplexing" is limited by the peak width (the "duty cycle bottleneck"), the direct inlet method according to the above may allow the steady state for practically unlimited "dwell time," which is technically advantageous to resolve the reporter ions for 10+ samples with high signal-to-noise, linking the "direct inlet" with the "plurality of three or more different biological samples".

The mass spectrometric method may be in some embodiments completely chromatography-free.

In such embodiments the method may avoid sample loss associated with solid-phase extraction and/or chromatographic columns. By enabling a workflow where the reaction mixture can be simply diluted in an acidified organic solvent and directly analyzed, the method preserves low-abundance analytes that might otherwise be irreversibly adsorbed to stationary phases during clean-up steps.

The term **'completely chromatography-free'** may refer to a workflow, where no liquid-phase separation based on the differential partitioning of analytes between a stationary phase and a mobile phase occurs between the steps of sample preparation and ionization. This may specifically exclude the use of high-performance liquid chromatography (HPLC), ultra-high-performance liquid chromatography (UHPLC), or micro-column separations or the like. By avoiding the fluidic complexities and temporal constraints of chromatography, the method may achieve a significant increase in sample-to-sample throughput and eliminates the carry-over effects and retention time shifts typically associated with column-based separations. Specifically, the phrase 'completely chromatography-free' may refer to a mass spectrometric workflow that avoids the use of high-performance liquid chromatography (HPLC), ultra-high-performance liquid chromatography (UHPLC), or similar column-based separations characterized by a temporal elution gradient based on differential partitioning between a mobile and a stationary phase. Crucially, this definition does not exclude, and specifically encompasses, the use of liquid-phase pre-processing, purification, or enrichment techniques that are non-chromatographic in nature. Such permissible steps include, but are not limited to, magnetobead-capturing, immunoprecipitation, or batch-mode solid-phase extraction, where the technical purpose is the isolation or concentration of a target analyte rather than the time-resolved separation of a complex mixture prior to ionization. Consequently, a method according to the present disclosure may be considered 'completely chromatography-free' even if the sample mixture undergoes an upfront bead-based enrichment step before being introduced into the ionization source via a direct inlet in a steady-state manner.

The chromatography-free nature of the mass spectrometric method may enable a transformative shift in clinical diagnostics and/or proteomics by fundamentally eliminating the most time-consuming and fragile components of the analytical stack. By bypassing and/or avoiding liquid chromatography (LC) and associated solid-phase extraction (SPE) steps, the total analysis time is reduced from hours (typically 30-120 minutes for column equilibration and gradient elution) to a timescale of seconds to minutes per sample. This approach provides the specific technical advantage of preventing sample loss associated with irreversible adsorption to stationary phases, thereby preserving the presence of low-abundance analytes. Furthermore, the removal of the LC system physically eliminates risks of capillary leaks, valve wear, and column clogging, while simultaneously removing the potential for carryover effects that lead to false positives in diagnostic settings. From a data processing perspective, being chromatography-free ensures that all analytes enter the ionization source under identical solvent conditions, thereby removing the need for computationally intensive retention time alignment of chromatograms and eliminating the errors associated with chromatographic drift

In some other embodiments, the method may not fully exclude a solid-phase extraction and/or chromatographic columns, for example in terms of sample preparation before being subjected to the method of the first aspect.

The fragmenting may be performed by collision with gas particles.

The fragmentation of separated labeled analyte ions via collision with gas particles may provide a highly controlled and reproducible means of releasing reporter ions for precise relative quantification. By utilizing collision-induced dissociation and/or higher-energy collisional dissociation, the kinetic energy of the accelerated ions is efficiently converted into internal energy through successive collisions with inert gas molecules (e.g., nitrogen or argon), leading to the predictable cleavage of the labile bond between the reporter and the balancer regions of the isobaric mass tag. This fragmentation mechanism is particularly advantageous for chromatography-free direct infusion because it enables high-speed MS/MS acquisition cycles that match the throughput of the ion mobility separation and static nano-electrospray stream. Furthermore, gas-phase fragmentation ensures that the resulting reporter ions (e.g., *m*/*z* 126.13 for TMTzero) exhibit a signal intensity that is directly proportional to the amount of analyte in each biological sample, providing the specific energy required to overcome the 'ratio compression' often associated with more complex matrices by selectively targeting ions purified in the preceding ion mobility stage.

The ion mobility separation step may provide an orthogonal dimension of purification by resolving ions based on their collision cross-section (CCS) before they enter the high-energy fragmentation zone. This ensures that the population of ions subjected to fragmentation is 'pre-purified' in the gas phase, preventing the contribution of chemical noise to the reporter ion channels and maintaining a linear relationship between signal intensity and analyte concentration across several orders of magnitude.

The step of separating the labeled analyte ions based on their ion mobility may comprise performing Trapped Ion Mobility Spectrometry (TIMS).

The implementation of TIMS for separating labeled analyte ions may provide unique advantages in sensitivity and/or duty cycle management for chromatography-free workflows. Unlike traditional drift-tube systems, TIMS typically utilizes an electric field to hold ions stationary against a moving buffer gas, allowing for the spatial accumulation and subsequent selective release of ions based on their Collisional Cross Section. This accumulation phase significantly may enhance the signal-to-noise ratio by concentrating the ion population before it reaches the fragmenter. The high-speed scan rates of TIMS may be natively compatible with the fast data acquisition requirements of the direct-inlet stream, maintaining analytical peak capacity without the need for temporal separation. Furthermore, TIMS may effectively resolve TMT-labeled peptides from singly charged chemical noise and isobaric interferences, which is the primary mechanism for eliminating ratio compression in quantitative measurements. This spatial separation may ensure that only high-purity precursor ion packets are subjected to fragmentation, resulting in reporter ion intensities that accurately reflect the true biological concentration of the analytes.

While TIMS is a preferred implementation for the spatial accumulation and selective release of ions, the method may alternatively or additionally utilize other ion mobility separation techniques to distinguish labeled analyte ions from background interferences. Suitable alternative methods include, but are not limited to, Drift Tube Ion Mobility Spectrometry (DTIMS), which separates ions based on their transit time through a uniform electric field in a buffer gas; Field Asymmetric Waveform Ion Mobility Spectrometry (FAIMS), which acts as a continuous filter by utilizing high-field asymmetric waveforms to selectively transmit ions of a specific mobility; and Traveling Wave Ion Mobility Spectrometry (TWIMS), which employs a dynamic electric field to propel ion packets through a gas-filled chamber. Each of these alternatives may serve the common technical objective of functioning as a gas-phase filter to remove singly charged background ions and isobaric interferences. By separating the labeled precursor ions from these contaminants based on their unique collisional cross-sections prior to fragmentation, these alternative separation modes may effectively mitigate the ratio compression effect and ensure high quantitative accuracy in a chromatography-free environment.

The mass spectrometric method may further comprise a step of filtering the labeled analyte ions based on their mass-to-charge ratio using a quadrupole mass filter prior to fragmenting the separated labeled analyte ions, wherein the quadrupole mass filter may be configured to transmit the labeled analyte ions and reject ions having mass-to-charge ratios different from the labeled analyte ions.

The inclusion of a quadrupole mass filtering step prior to fragmentation may provide an additional, orthogonal layer of precursor purification that significantly enhances quantitative precision. While the ion mobility separator may act as a gas-phase filter based on molecular shape and charge, the quadrupole mass filter may specifically target the mass-to-charge (*m*/*z*) ratio of the labeled analyte ions. By narrowing the isolation window to transmit only the precursor of interest while rejecting off-target ions with different *m*/*z* values, the system may further reduce the potential for co-fragmentation. This synergy between ion mobility and mass filtering may be especially advantageous in chromatography-free direct infusion, where the 'precursor ion population' is inherently more complex. The technical effect may be a drastic reduction in chemical noise and the total elimination of interfering signals that could otherwise lead to 'ratio compression', thereby ensuring that the detected reporter ions are derived exclusively from the target analyte.

While a quadrupole mass filter may be preferred for precursor isolation, the method may alternatively employ other mass selection components to isolate labeled analyte ions prior to fragmentation. Suitable alternative methods may include: Linear Ion Traps (LIT): These utilize oscillating radio frequency (RF) fields to trap and selectively eject ions based on their *m*/*z,* offering high sensitivity and the ability to perform multiple stages of fragmentation (MSⁿ); Time-of-Flight (TOF) Gating (Timed Ion Selector): In some architectures, high-speed 'gates' can be used to deflect unwanted ions based on their flight time, effectively selecting a specific *m*/*z* window for subsequent fragmentation; Orbitrap Mass Analyzers: These can be used for the ultra-high-resolution selection and trapping of specific precursor masses, providing exceptional specificity in identifying isobaric species before they enter the fragmenter module; Electrostatic Ion Traps:
These offer similar mass-selective trapping capabilities to LITs but rely on static electric fields, providing a compact alternative for precursor isolation within the mass spectrometer system.

Linear Ion Traps may be very useful for low-abundance analytes because they can accumulate ions before fragmentation, similar to how TIMS accumulates ions spatially. TOF Gating may be naturally compatible with the Time-of-Flight (TOF) mass spectrometers. Orbitraps may provide a high resolution to distinguish between nearly identical precursor masses that even ion mobility might not fully resolve.

The first biological sample may be a patient sample and the second biological sample may be a control sample, and determining the relative quantity may comprise comparing the measured signal intensity of the reporter ion from the patient sample to the measured signal intensity of the reporter ion from the control sample; or the first biological sample may be a first patient sample and the second biological sample may be a second patient sample of the same patient or a different patient, and determining the relative quantity may comprise comparing the measured signal intensity of the reporter ion from the first patient sample to the measured signal intensity of the reporter ion from the second patient sample.

Comparing a patient sample against a control sample or against another patient sample enables high-throughput clinical decision-making with inherent, physical normalization. In the patient-versus-control embodiment, a pooled reference or a control sample, e.g. a healthy control sample, is labeled with a specific isobaric mass tag to serve as a common internal standard. Because the patient and control samples are ionized, spatially accumulated, and fragmented simultaneously within the same spectral acquisition cycle, any global fluctuations in instrument performance and/or ionization efficiency affect both samples proportionally. This cancels out potential errors and allows the ratio of measured signal intensities to accurately reflect the true biological up- or down-regulation of the analyte. In the patient-versus-patient sample comparison, the method allows for direct comparative screening of different individuals and/or longitudinal monitoring of the same patient (e.g., pre-treatment versus post-treatment). This eliminates the need for external calibration curves for every run, drastically reduces total analysis time per patient by leveraging multiplexing, and ensures that all patient results are normalized against an identical reference without run-to-run variations.

The measuring of the signal intensities may comprise using a Time-of-Flight (TOF) mass spectrometer.

The utilization of a Time-of-Flight (TOF) mass spectrometer may provide a degree of high-speed spectral acquisition and high resolution beneficial to resolve and/or quantify multiple reporter ions within a single, rapid scan cycle. Because a TOF analyzer determines the mass-to-charge (*m*/*z*) ratio based on the differential flight times of ions across a field-free drift region, it is inherently capable of recording a plurality of time-of- flight transients at frequencies exceeding 1 kHz. This extremely high acquisition rate may be highly compatible with the steady-state signal produced by the static nanoESI source and the millisecond-scale pulses from the ion mobility separator, allowing for the digital accumulation of thousands of transients into a single, high-fidelity mass-resolved spectrum.

One major technical advantage is that the TOF detector can distinguish multiple reporter ions (e.g., from TMT-labeled patient and control samples) simultaneously within each acquisition cycle, allowing that the relative quantity of analytes may be determined from an identical population of ions. This may eliminate the scanning overhead associated with slower analyzers and may provide a high-duty cycle that maximizes the signal-to-noise ratio, which is important for quantifying low-abundance biomarkers in complex clinical matrices without prior chromatographic separation.

While a Time-of-Flight (TOF) mass spectrometer may be preferred for measuring signal intensities due to its high acquisition speed and native compatibility with high-repetition-rate transients, the mass spectrometric method may alternatively employ other high-resolution mass analyzers to resolve and quantify the reporter ions. Suitable alternative mass spectrometers may include Orbitrap mass analyzers, which typically utilize Fourier transform-based detection of ion frequencies in an electrostatic field to provide ultra-high mass resolution and high dynamic range, thereby facilitating the separation of closely spaced reporter ions in complex multiplexed experiments. Fourier Transform Ion Cyclotron Resonance (FT-ICR) mass spectrometers may also be utilized, offering exceptional mass accuracy and resolution by measuring the cyclotron frequency of ions in a strong magnetic field. Additionally, or alternatively, electrostatic ion traps or magnetic sector analyzers may be implemented to measure the signal intensities of the reporter ions at their respective mass-to-charge ratios.

Each of these alternatives may serve the common technical objective of accurately detecting the mass-resolved signals of the released reporter ions to determine the relative quantity of the at least one analyte present in the first biological sample relative to the second biological sample, ensuring that the quantitative data is extracted with high precision even when bypassing chromatographic separation.

According to another aspect of the invention, which may be considered a second aspect, a mass spectrometer system for determining a relative quantity of at least one analyte present in a first biological sample relative to the quantity of the same at least one analyte present in a second biological sample comprises: a sample supply interface configured to introduce a stream of a sample mixture into an ionization source by means of a direct inlet, wherein the sample mixture comprises the at least one analyte from the first biological sample labeled with a first isobaric mass tag and the at least one analyte from a second biological sample labeled with a second isobaric mass tag different from the first isobaric mass tag; the ionization source configured to ionize the stream of the sample mixture to generate labeled analyte ions; an ion mobility separator positioned downstream of the ionization source and configured to separate the labeled analyte ions from other components of the sample mixture based on their ion mobility; a fragmenter module configured to fragment the separated labeled analyte ions to release reporter ions from the first and second isobaric mass tags; a detector configured to measure signal intensities of the reporter ions at their respective mass-to-charge ratios; and a controller configured to determine the relative quantity of the at least one analyte present in the first biological sample relative to the second biological sample based on the measured signal intensities.

The mass spectrometer system has advantages and technical effects as described for corresponding features of the method.

The detector may comprise a Time-of-Flight (TOF) detector; and/or the controller may be configured to distinguish the reporter ions from the first isobaric mass tag and the second isobaric mass tag based on their differential flight times to the detector, such that the reporter ions are detected sequentially within a single spectral acquisition cycle; and/or the controller may be configured to orchestrate the simultaneous analysis by: a) recording a plurality of time-of-flight transients in which the reporter ions of the first and second isobaric mass tags arrive at the TOF detector at distinct times based on their differential flight times; b) digitally accumulating said transients into a final mass-resolved spectrum; and c) calculating the ratio of the signal intensities of the reporter ions extracted from said final mass-resolved spectrum.

The mass spectrometer system may utilize a feedback-controlled acquisition logic wherein the digital accumulation of spectra may be performed online during the introduction of the constant sample stream. This may provide the specific technical advantage of 'sensitivity-on-demand', where the controller monitors the statistical quality of the accumulating final mass-resolved spectrum in real-time. The measurement may be concluded only once a pre-defined statistical threshold-specifically a coefficient of variance (CV) and/or a signal-to-noise ratio (SNR) of about 20%-is achieved for the reporter ion intensities. This mechanism may ensure that the relative quantification is robust against varying initial sample concentrations, preventing over-acquisition of high-abundance analytes while ensuring sufficient measurement time for low-abundance biomarkers, thereby standardizing data quality across a diverse clinical cohort.

The system may be configured to orchestrate multiple hardware components operating at vastly different frequencies, specifically synchronizing the ion mobility separation with the mass detector. The controller may manage the ion mobility separation at a frequency of approximately 3 Hz to 25 Hz, while the recording of mass-resolved spectra by the Time-of-Flight (TOF) detector is performed at frequencies exceeding 1 kHz. This nested timing framework may allow the system to capture hundreds of individual mass-resolved transients within a single ion mobility pulse. The resulting technical advantage may be the generation of a high-density, multi-dimensional data map that resolves isobaric interferences in the gas phase without the need for the minutes-long temporal separation typically provided by liquid chromatography.

The term **"nested timing framework"** may refer to a synchronized operational mode where multiple hardware components operate at different frequencies such that higher-frequency events occur within the duration of lower-frequency events. Specifically, this may involve recording a plurality of high-frequency mass-resolved transients (e.g., exceeding 1 kHz) within the timeframe of a single, lower-frequency ion mobility separation pulse (e.g., 3 Hz to 25 Hz).

The nested timing framework may be characterized by a frequency ratio between the detector and the ion mobility separator of at least 40:1, more specifically at least 100:1, allowing for the reconstruction of high-resolution ion mobility heat maps without compromising mass spectral fidelity.

To manage the extreme data influx from the continuous nanoESI stream, the system may employ a specialized two-processor architecture for digital accumulation. A first data processor, integrated with the detector, may perform the "pre-summing" of native mass-resolved spectra, which are then passed to a second, separate data processor for final "summing" into the final mass-resolved spectrum. This distributed processing may provide the technical effect of preventing the main system controller from being overwhelmed by raw high-frequency data transients. The advantage of this tiered approach may be that it enables a high-duty cycle and real-time data processing, ensuring that the system can maintain high resolution and quantitative precision even during the rapid analysis of complex, unpurified clinical samples.

Unlike traditional LC-MS systems that utilize fixed acquisition windows, the system controller may be configured to manage acquisition dynamically based on online data monitoring. The controller may monitor the statistical quality of the accumulating spectrum in real-time and may be programmed to conclude the measurement only when a pre-defined statistical threshold, such as a coefficient of variance (CV) and/or for example, a signal-to-noise ratio (SNR) of approximately 20% may be reached. This "sensitivity-on-demand" may provide the technical advantage of standardizing data quality across a diverse clinical cohort, effectively eliminating "under-sampling" of low-concentration patient samples while optimizing instrument throughput by preventing unnecessary over-acquisition of high-abundance analytes.

The term **"sensitivity-on-demand"** may refer to an automated acquisition logic where the controller monitors the data quality of the accumulating spectrum in real-time. The measurement duration may not be fixed but may be dynamically determined by the system, concluding only when a pre-defined statistical threshold, such as a specific Coefficient of Variance (CV) or Signal-to-Noise Ratio (SNR), is achieved.

The ion mobility separator, specifically in the implementation of a Trapped Ion Mobility Spectrometry (TIMS) device, may utilize a unique spatial management of ions. Labeled analyte ions may first be spatially accumulated in an accumulation tunnel with a volume of approximately 1 cm³ to 70 cm³ before being selectively released into the separation tunnel. This accumulation phase may provide the technical effect of concentrating the ion population into discrete, high-density packets prior to fragmentation and detection. The primary advantage may be a significant enhancement of the signal-to-noise ratio, which may allow the system to detect and quantify low-abundance biomarkers within the complex chemical backgrounds of "dirty" matrices that would otherwise remain below the detection limit in standard direct-infusion setups.

The system architecture may be designed to transport the mixed patient and control samples through the analytical stages as a single, co-existing ion population. The labeled analyte ions from both the first and second biological samples may be processed simultaneously through the ion mobility separator and into the fragmenter module as a combined precursor population. This may allow that both samples are subjected to identical electrical fields, gas pressures, and collision energies at the exact same moment. The technical advantage may be a physical normalization at the hardware level that cancels out any potential errors derived from instrument drift or ionization fluctuations, ensuring that the calculated ratios represent true biological differences rather than hardware-induced variance.

The first and second isobaric mass tags may render the analytes from the first and second biological samples isobaric and/or isostructural, such that the system may be configured to transport the labeled analyte ions through the ion mobility separator and into the fragmenter module simultaneously as a combined precursor ion population.

The step of performing of the ion mobility separation may be performed in a pulsed manner over first separation time spans. Filtering by the quadrupole mass filter may be performed in a pulsed manner over second separation time spans. Recording the mass-resolved spectra may be performed in a pulsed manner over recording time spans, wherein the time spans are related such that the first separation time span is greater than the second separation time span, and the second separation time span is greater than the recording time span.

In accordance with certain embodiments of the system, the ion mobility separator and the quadrupole mass filter may operate in a nested, pulsed timing framework to maximize duty cycle and sensitivity. Specifically, the ion mobility separation may be performed over a first separation time span (e.g., milliseconds), while the quadrupole filtering may occur over a shorter second separation time span, and the final TOF recording may occur over even shorter recording time spans. This hierarchical timing may allow that the high-frequency TOF transients (exceeding 1 kHz) are synchronized with the lower-frequency pulses of the TIMS device (about 3 Hz to 25 Hz), effectively 'mapping' the ion mobility dimension onto the mass-to-charge dimension without data loss. Furthermore, the use of an accumulation tunnel with a volume of approximately 1 cm³ to 70 cm³ may allow for the spatial concentration of labeled analyte ions before they are released into the separation tunnel, which may have an effective separation length of about 5 cm to 1.5 m. This specific physical configuration may provide the analytical power suitable to resolve isobaric interferences in a chromatography-free environment.

The term **"effective separation length"** may refer to the path length over which ions are subjected to the mobility-separating forces within the ion mobility separator. In a trapped system such as TIMS, this length may relate to the dimensions of the separation tunnel where ions are selectively released against a gas flow.

The pulsed separation and/or recording time spans may refer to the synchronized timing intervals where the ion mobility separation (first time span), quadrupole mass filtering (second time span), and/or detector recording (recording time span) may occur. These spans may be nested such that multiple mass-resolved spectra are recorded within a single ion mobility pulse to ensure multidimensional data density.

The ion mobility separation may be performed at a frequency of about 3 Hz to 25 Hz, and the recording of the mass-resolved spectra is performed at a frequency exceeding 1 kHz.

The separating the labeled analyte ions may comprise spatially accumulating the labeled analyte ions in an accumulation tunnel having a volume of approximately 1 cm³ to 70 cm³ prior to releasing them into a separation tunnel.

The accumulation tunnel and/or separation tunnel may refer to the discrete physical stages within an ion mobility separator, such as a TIMS device. The "accumulation tunnel" may be configured to spatially trap and concentrate ions using an electric field against a gas flow, while the "separation tunnel" may be configured to selectively release those ions based on their collisional cross section.

The separating of the labeled analyte ions may be performed in an effective separation length of about 3 cm to 8 m, specifically about 5 cm to 1.5 m.

The mass spectrometric method may further comprise a step of pre-processing the first and/or the second biological sample prior to mixing, wherein said pre-processing comprises collecting the at least one analyte from a body fluid, specifically serum or plasma, by magnetobeads-capturing.

Prior to mixing, the biological samples may undergo a pre-processing step involving magnetobead-capturing. This step may specifically refer to the collection of the at least one analyte (e.g., a specific protein or peptide) from a complex body fluid like serum and/or plasma using functionalized magnetic beads (denoted magnetobeads). This pre-processing may serve as a high-speed, liquid-phase alternative to traditional chromatography, allowing for the enrichment of target analytes and the removal of bulk matrix proteins before the labeling reaction. When combined with the chromatography-free direct infusion method, this may ensure that the mass spectrometer receives a sample population that is both enriched for the analytes of interest and compatible with the high-throughput requirements of large-scale biomarker screening.

Magnetobead-capturing may refer to a pre-processing technique where target analytes are selectively bound to functionalized magnetic particles within a liquid biological matrix. This may allow for the physical isolation and enrichment of proteins and/or peptides from complex fluids like serum and/or plasma prior to the labeling reaction, acting as a non-chromatographic purification step.

The first and/or the second biological sample may comprise serum samples separated from whole blood.

The step of measuring the signal intensities may comprise: i) detecting a plurality of raw spectral data sets; ii) generating a plurality of native mass-resolved spectra from the raw data; iii) pre-summing the native mass-resolved spectra to obtain at least two pre-summed spectra using a first data processor integrated with the detector; and iv) summing the at least two pre-summed spectra to obtain the final mass-resolved spectrum using a second data processor different from the first data processor.

To handle the extreme data rates generated by a TOF detector operating at frequencies exceeding 1 kHz, the system may employ a tiered data processing architecture. A first data processor, integrated directly with the detector, may perform 'pre-summing' of native mass-resolved spectra to reduce the raw data volume without losing spectral information. Subsequently, a second data processor may perform the final 'summing' of these pre-summed spectra to obtain the final mass-resolved spectrum used for quantification. This distributed processing approach may provide the technical effect of realizing and/or supporting real-time, high-resolution data acquisition from a continuous spray while maintaining a high duty cycle, which is beneficial for achieving the precision required in routine clinical diagnostics.

The "pre-summing" may refer to the initial integration of raw spectral transients by a first data processor (e.g., an FPGA or local detector hardware) to reduce data throughput. "Summing" may refer to the subsequent aggregation of these pre-summed data sets by a second, typically more powerful data processor to generate the final mass-resolved spectrum for quantification.

The accumulation of the spectra may be performed online during the introduction of the stream, specifically wherein the method may further comprise stopping the measurement when a coefficient of variance or a signal-to-noise ratio of the accumulated spectrum reaches a pre-defined threshold value, specifically about 20%.

The stopping may be software-controlled stopping and may improve the mass spectrometer's operation, for example, by optimizing duty cycle and/or preventing emitter clogging (rather than just being a "statistical" rule).

The method may comprise dynamically controlling the duration of the introducing and/or ionizing step by: (i) monitoring, by a controller, a standard deviation or a coefficient of variation (CV) of the measured signal intensities of the reporter ions in real-time; and (ii) automatically generating a control signal to terminate the accumulation of ions for the at least one analyte in the mass-to-charge analyzer upon the CV reaching a pre-defined threshold, thereby optimizing the duty cycle of the mass spectrometer and/or reducing sample-induced contamination of the ionization source.

The coefficient of variance (CV) and/or Signal-to-Noise Ratio (SNR) threshold may refer to a pre-defined statistical quality metric used by the controller to determine the completion of a measurement. A threshold of "about 20%" may indicate that the digital accumulation of spectra continues dynamically until the relative standard deviation of the reporter ion intensities falls below this value, ensuring quantitative precision regardless of initial analyte concentration.

The step of ionizing the stream may comprise using a static nano-electrospray emitter at a flow rate of about 200 nL/min to about 1000 nL/min.

The step of measuring the signal intensities may comprise performing a data-dependent acquisition (DDA) and/or a targeted analysis directly from an unseparated eluate of the sample mixture.

Targeted analysis may refer to a mass spectrometric acquisition mode where the instrument is programmed to monitor only specific, pre-defined precursor *m*/*z* values and their corresponding reporter ions. This may be distinct from data-dependent acquisition (DDA), as it prioritizes known biomarkers of interest directly from the unseparated sample mixture

The step of separating the labeled analyte ions based on their ion mobility may be configured to separate the labeled analyte ions from singly charged background noise, including solvent ions and lipids, thereby reducing ratio compression in the determined relative quantity.

The step of separating the labeled analyte ions based on their ion mobility may be configured to separate isobaric precursor masses prior to the fragmenting step.

The method may be performed such that all samples in the sample mixture are ionized under identical conditions at the same time, thereby eliminating run-to-run ionization variance.

The step of determining of the relative quantity may be performed without retention time alignment of chromatograms.

The first and/or second labeling reagents may be selected from the group consisting of TMTzero reagents having a mass reporter ion at *m*/*z* 126.13 and TMT-Sixplex reagents having a mass reporter ions at *m*/*z* 126.13, 127.13, 128.13, 129.13, 130.14 and 131.14.

Regarding the detection of the reporter ions, it is to be understood that the values ranging from *m*/*z* 126.13 to 131.14, represent the theoretical excact *m*/*z* values for the six reporter ions. It will be appreciated by the skilled person that experimentally determined values may slightly deviate from the theoretical exact masses of the corresponding reporter ions due to factors such as standard instrument calibration variations and/or inherent measurement tolerances.

In one embodiment the first and/or second labeling reagents are selected from the group consisting of TMT-Sixplex reagents TMT-126, TMT-127, TMT-128, TMT-129, TMT-130 and TMT-131 with the following formula

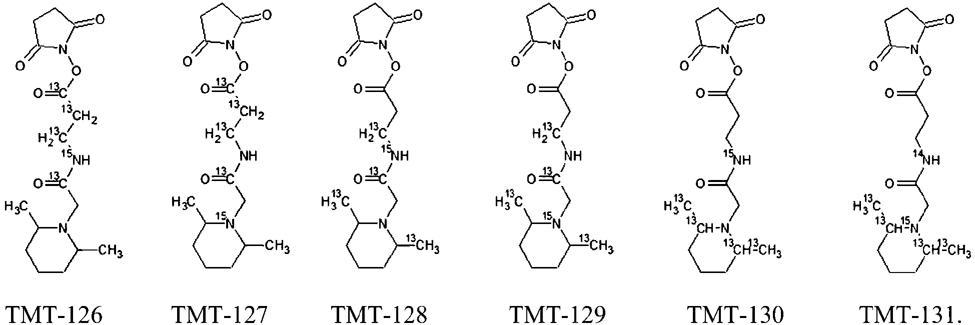

The term **"about" or "approximately"** in the context of numerical values or ranges may refer to a variation of 10\% of the stated value, unless the specific context indicates a different tolerance (such as the specific chemical concentrations defined in the acidified organic solvent).

Below are described some specific optional embodiments and features of the method according to the first aspect or embodiments thereof.

In some embodiments, the mass spectrometric method of the first aspect or an embodiment thereof may be suited for detecting one or more analytes in a biological sample solution and may comprise the steps:
providing a constant stream of ionized sample molecules comprising the analyte from the biological sample solution and an analyte-specific internal standard including electrospray ionization (ESI) and/or nano-electrospray ionization (nano-ESI);
performing a spatial accumulation of the analyte and the analyte-specific internal standard and performing at least partially a first separation of the analyte and the analyte-specific internal standard from other components of the ionized sample molecules based on the size and/or shape of the ionized molecules;
performing at least partially a second separation of the analyte and the analyte-specific internal standard from remained other components of the ionized sample molecules based on the mass to charge ratio of the ionized molecules;
fragmenting the ionized analyte into ionized analyte fragments and the ionized analyte-specific internal standard into ionized analyte-specific internal standard fragments by collision with particles of a gas phase;
providing a stream comprising the ionized analyte fragments and the ionized analyte-specific internal standard fragments on a detection mass spectrometer; and
recording with the detection mass spectrometer at least two pre-summed mass-resolved spectra and summing the at least two pre-summed mass-resolved spectra to obtain a final mass-resolved spectrum.

The step of providing the constant stream of ionized sample molecules may be free of a generation and/or a providing of a pulsed stream of ionized sample molecules.

The step of recording of the pre-summed mass-resolved spectra may comprise a pre-summing of native mass-resolved spectra to obtain the pre-summed mass-resolved spectra, which is performed by a first data processor; and
the step of summing of the at least two pre-summed mass-resolved spectra to obtain a final mass-resolved spectrum may be performed by a second data processor different from the first data processor, specifically wherein the first data processor may correspond to a data processor comprised by the detection mass spectrometer and the second data processor may correspond to an external data processor; and/or wherein at least one of the at least two pre-summed mass-resolved spectra may be recorded at a frequency between about 20kHz and about 100Hz, specifically between about 5kHz and about 100Hz; and/or
wherein the step of recording the at least two pre-summed mass-resolved spectra may be repeatedly performed during a time span of about 5s to about 1h, specifically during a time span of about 15s to about 10 minutes and more specifically during a time span of about 30s to about 2 minutes; and/or
wherein the at least two pre-summed mass-resolved spectra may correspond to about 5 to about 500 pre-summed mass-resolved spectra which are summed to obtain a final mass-resolved spectrum.

The step of providing the constant stream of ionized sample molecules and the analyte-specific internal standard may comprise, prior to the ESI and/or nano-ESI, providing of the sample solution as a pre-processed sample solution including separating a serum from a whole blood sample, collecting the analyte from the serum by at least one of the following: magnetobeads-capturing, immunoassay methods, and/or adding the analyte-specific internal standard; and/or
the constant stream of ionized sample molecules and the analyte-specific internal standard and/or the stream of ionized analyte fragments and ionized analyte-specific internal standard fragments may be generated for a time span of up to about 15 minutes, specifically at a pre-processed sample solution volume of about 1nL/min to about 1000nL/min, specifically about 200nL/min to about 500nL/min, more specifically about 250nL/min to about 350nL/min.

The step of summing the at least two pre-summed mass-resolved spectra to obtain the final mass-resolved spectrum may be completed, stopped and/or considered sufficiently sensitive when a coefficient of variance and/or an S/N ratio reaches a pre-defined threshold value, specifically the threshold value may correspond to about 20% .

The step of performing the first separation may comprise ion mobility spectrometry; and/or
the performing of the second separation may comprise quadrupole mass spectrometry.

The detection mass spectrometer may comprise and/or may be based on at least one of the following: time-of-flight (TOF), Triple Quad (QQQ), Ion Trap, an Orbi-Trap mass spectrometry, sector field MS, specifically wherein the detection mass spectrometer is combined with an ionization unit comprising at least one of the following: NanoSpray, ESI, APCI.

The step of performing the second separation may be repeated at least one time.

The step of providing of the constant stream of the analyte and the analyte-specific internal standard may be performed over a providing time span and the step of performing of the spatial accumulation of the analyte and the analyte-specific internal standard may be performed over an accumulation time span.

The step of performing the first separation of the analyte and the analyte-specific internal standard may be performed in a pulsed manner over first separation time spans.

The step of performing the second separation of the analyte and the analyte-specific internal standard may be performed in a pulsed manner over second separation time spans.

The step of fragmenting of the ionized analyte into ionized analyte fragments and the analyte-specific internal standard into ionized analyte-specific internal standard fragments may be performed in a pulsed manner over fragmenting time spans.

The step of recording with the detection mass spectrometer at least two pre-summed mass-resolved spectra may be performed in a pulsed manner over recording time spans, specifically
wherein (t0a) > (t1) > (t2) = (t3) > (t4) or (t0a) > (t1) > (t2) > (t3) > (t4), and/or
wherein the providing time span may range between about 15s and about 30 minutes, each of the first separation time spans may range between about 40ms and about 300ms, each of the second separation time spans may range between about 1ms and about 40ms, each of the fragmenting time spans may range between about 1ms and about 40ms and each of the recording time spans may be shorter than 1ms, specifically may range between about 1ms and about 50µs.

The step of providing the constant stream of the analyte and the analyte-specific internal standard may be performed constantly.

The step of performing the first separation of the analyte and the analyte-specific internal standard may be performed at a frequency of about 25Hz to about 3Hz.

The step of performing the second separation of the analyte and the analyte-specific internal standard may be performed at a frequency of about 1kHz to about 25Hz.

The step of fragmenting the ionized analyte into ionized analyte fragments and the analyte-specific internal standard into ionized analyte-specific internal standard fragments may be performed at a frequency of about 1kHz to about 25Hz.

The step of recording with the detection mass spectrometer of at least two pre-summed mass-resolved spectra may be performed at a frequency exceeding about 1kHz.

Below are described some specific optional embodiments and features of the system according to the second aspect or embodiments thereof.

The mass spectrometer system may be suited for detecting at least one analyte in a biological sample solution and may comprise:
a sample supply module, comprising a sample pre-processor and an ionizer, the sample supply module being configured to provide a constant stream of ionized sample molecules including the analyte from a sample solution and an analyte-specific internal standard, wherein the ionizer is based on electrospray ionization (ESI) and/or nano-electrospray ionization (nano-ESI);
a first selectivity enhancer module configured to perform a spatial accumulation of the analyte and the analyte-specific internal standard and to perform at least partially a first separation of the analyte and the analyte-specific internal standard from other components of the ionized sample molecules based on the size and/or shape of the ionized molecules;
a second selectivity enhancer module configured to perform at least partially a second separation of the analyte and the analyte-specific internal standard from remained other components of the ionized sample molecules based on the mass to charge ratio of the ionized molecules;
a fragmenter module configured to fragment the ionized analyte into ionized analyte fragments and the analyte-specific internal standard into ionized analyte-specific internal standard fragments by collision with particles of a gas phase;
a detection module comprising a detection mass spectrometer with a first data processor and being configured to record at least two pre-summed mass-resolved spectra from a stream of the ionized analyte fragments and the ionized analyte-specific internal standard fragments; and
a controller module comprising a second data processor configured to sum the at least two pre-summed mass-resolved spectra to obtain a final mass-resolved spectrum.

The sample pre-processor may be configured to collect the analyte molecules from the serum by at least one of the following: magnetobeads-capturing and/or immunoassay methods.

The first selectivity enhancer module may comprise an ion mobility spectrometer.

The second selectivity enhancer module and/or the fragmenter module may comprise a quadrupole mass spectrometer and/or a gas-filled chamber.

The mass spectrometer system and/or the mass spectrometer may comprise at least one of the following: time-of-flight (TOF), Triple Quad (QQQ), Ion Trap, an Orbi-Trap mass spectrometry.

The first selectivity enhancer module may provide an effective length configured for the first separation of the analyte and the analyte-specific internal standard from other components of the ionized sample molecules of about 3 cm to about 8 m, specifically of about 4 cm to about 3 m, more specifically of about 5 cm or of about 1.5 m, specifically wherein the first selectivity enhancer module comprises and/or corresponds to a TIMS cartridge with an accumulation tunnel and a TIMS tunnel, wherein the TIMS tunnel provides the effective length configured for the first separation.

### Detailed Description of the Invention

In the following, some example embodiments will be described in detail, wherein the invention should not be understood to be limited to the examples and embodiments described. The following examples and embodiments are provided to aid the understanding of the present invention, the true scope of which is set forth in the appended claims. Single features being described in a particular embodiment may be arbitrarily combined, given that they are not excluding each other. In addition, different features, which are provided together in the example embodiments, are not to be considered restrictive to the invention or the embodiment.

### Exemplary Embodiment

Experiments were carried out on a commercial timsTOF HT (Bruker Daltonics) mass spectrometer. Ionization of the analytes was performed with a Triversa Nanomate (Advion Interchim). To experimentally validate the performance of the method according to the present invention, a multiplex analysis was performed using the model peptide Leucine Enkephalin. Leucine Enkephalin has the following structural formula:

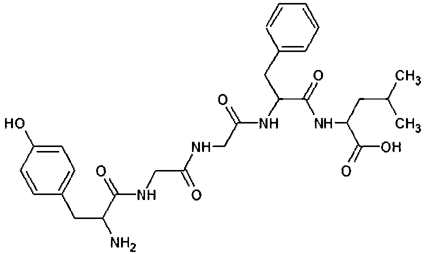

In a first step, a calibration series was prepared by providing six independent samples containing defined concentrations of the peptide Leucine Enkephalin (Cal1: 90 µM, Cal2: 180 µM, Cal3: 360 µM, Cal4 900 µM, Cal5: 1800 µM, Blank: 0 µM), which has a molecular weight of 555.27 g/mol. To label the samples, a TMT-Sixplex set (TMT-126,TMT-127, TMT-128, TMT-129, TMT-130, TMT-131) as labeling reagents set was utilized.

The labeling reagents have the following structural formula

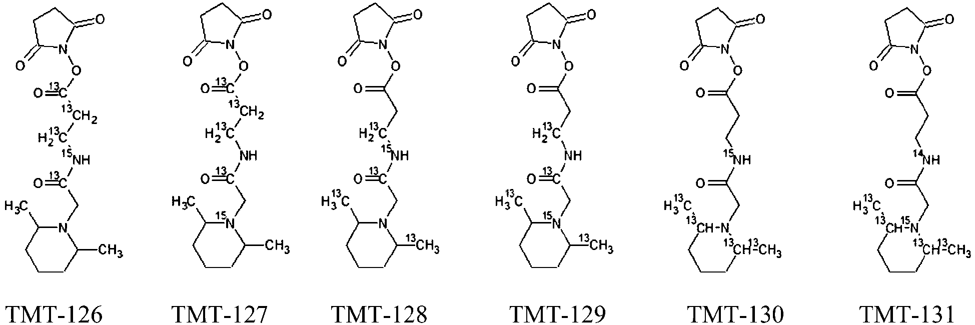

The labeling reagents were equilibrated to room temperature and subsequently dissolved by adding about 41 µl of anhydrous acetonitrile per about 0.8 mg reagent vial, followed by occasional vortexing for about 5 minutes. For the labeling reaction, about 41 µl of the prepared TMT reagent solution (TMT-126 to TMT-131) was added to about 100 µl of the respective Leucin-Enkephaline analyte sample, which was provided in a TEAB buffer. The reaction was carried out for one hour at about 25 °C under constant shaking to form the analyte labeled with respective isobaric mass tag. Optionally, the reaction may be quenched by adding about 8 µl of about 5% hydroxylamine and incubating for about 15 minutes to deactivate any excess reagent.

The structural formula of the analyte labeled with the isobaric mass tag (shown only for the labeling reagent TNT-126) is:

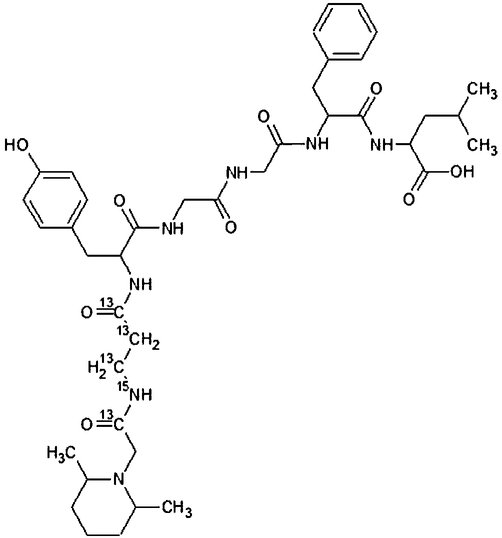

Following the labeling and the provision of six samples comprising the analyte labeled with the isobaric mass tags, each of the six samples was diluted about 1:10 with an acidified organic solvent consisting of about 90 vol% acetonitrile (ACN), about 0.1 vol% formic acid (FA) and about 9.9 vol% H₂O. From these dilutions, about 10 µl each were taken and mixed in a common sample vessel to form the sample mixture.

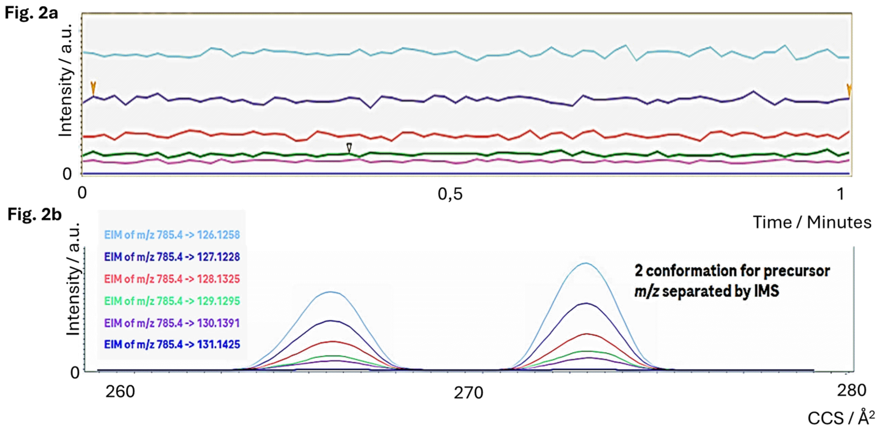

The mass spectrometric analysis of this sample mixture was performed directly and without any prior chromatographic cleanup using a TOF mass spectrometer. The sample introduction was realized via a static nanoESI direct inlet, whereby the stream of the sample mixture was continuously ionized in positive mode for about 1 minute. The extracted ion chromatograms (EIC) of the selected MS/MS transitions over a one-minute interval are shown in **Fig.2a****.**

The labeled analyte ions, exhibiting a mass-to-charge ratio (*m*/*z*) of approximately 785.44, were subsequently processed using Trapped Ion Mobility Spectrometry (TIMS). The ion mobility separation allowed for the resolution of different conformations of the precursor ion based on their collisional cross-section (CCS), thereby achieving an effective gas-phase purification of the target analyte population from background interferences. The separation of precursor m/z ratios is shown in **Fig. 2b****.**

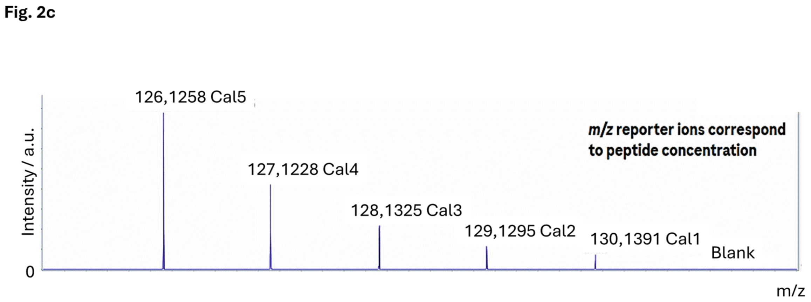

Following the ion mobility isolation, the labeled analyte ions were fragmented using Collision Induced Dissociation to release the reporter ions. The signal intensities were measured at the characteristic m/z ratios of 126.1258, 127.1228, 128.1325, 129.1295, 130.1391, and 131.1425. The mass spectrum identifying the six distinct TMT reporter ions that was used for relative quantification is shown in **Fig.2c****.**

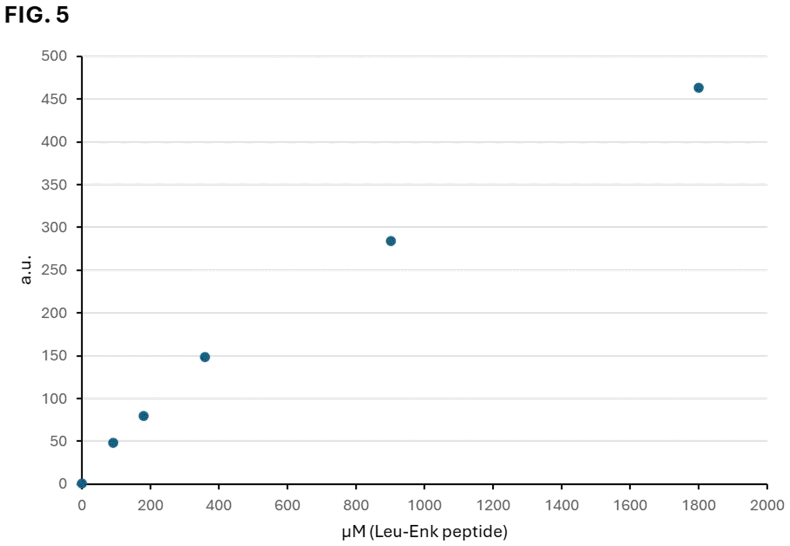

The experiment demonstrated a stable steady-state signal over a measurement duration of one minute. The resulting signal intensities of the reporter ions correlated very well with the initially employed peptide concentrations as shown in **Fig. 5****.** This demonstrates that the use of the specific acidified organic solvent in combination with ion mobility filtration enables accurate relative quantification while effectively mitigating negative effects such as ratio compression, despite the complete omission of liquid chromatography.

### Description of the Figures

**FIG. 1** is a flow chart illustrating a method for determining a relative quantity of at least one analyte present in a first biological sample according to an embodiment;
**FIG. 2a** depicts an extracted ion chromatogram illustrating the steady-state signal of a sample mixture comprising six independently TMT-labeled concentrations of an analyte obtained by using a method according to an embodiment;
**FIG. 2b** is a mobilogram showing the separation of the labeled analyte ions based on their ion mobility obtained by using a method according to an embodiment;
**FIG. 2c** is a mass spectrum illustrating the detection of six distinct TMT reporter ions released from the fragmented analyte ions obtained by using a method according to an embodiment;
**FIG. 3** is a methodical flow chart including functions of a mass spectrometer system according to an embodiment;
**FIG. 4** is a schematic drawing of a mass spectrometer system according to another embodiment; and
**FIG.5** is a correlation plot illustrating the quantitative performance of a method according to an embodiment.

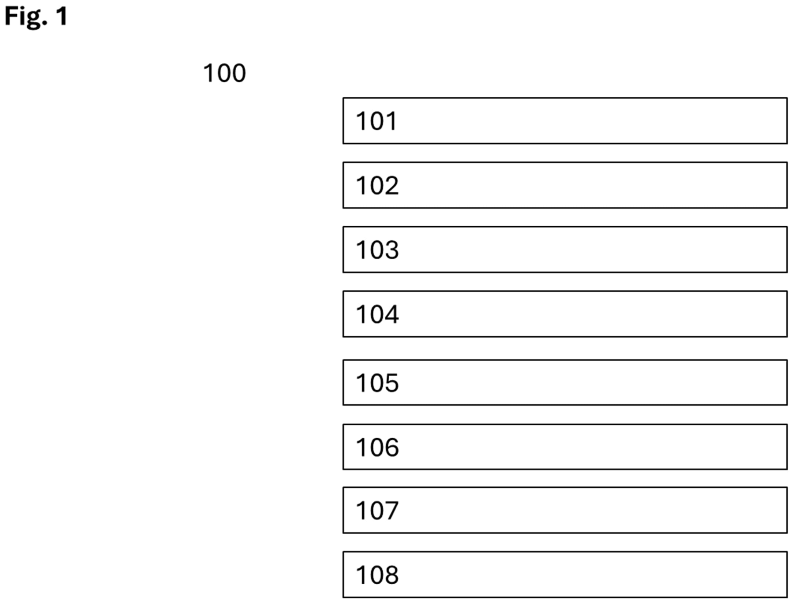

**FIG. 1** is a flow chart illustrating a mass spectrometric method 100 for determining a relative quantity of at least one analyte present in a first biological sample relative to the quantity of the same at least one analyte present in a second biological sample, the method comprising the steps of: providing 101 the first biological sample comprising the at least one analyte labeled with a first isobaric mass tag; providing 102 the second biological sample comprising the at least one analyte labeled with a second isobaric mass tag different from the first isobaric mass tag; mixing 103 the first biological sample with the second biological sample to obtain a sample mixture; introducing 104, by direct inlet, a stream of the sample mixture into an ionization source; ionizing 105 the stream of the sample mixture to generate labeled analyte ions of the at least one analyte labeled with the first and labeled with the second isobaric mass tag; separating 106 the labeled analyte ions from other components of the sample mixture based on their ion mobility; fragmenting 107 the separated labeled analyte ions to release reporter ions from the first and second isobaric mass tags; and measuring 108 signal intensities of the reporter ions at their respective mass-to-charge ratios and determining therefrom the relative quantity of the at least one analyte present in the first biological sample relative to the second biological sample based on the measured signal intensities.

**FIG. 2a** depicts an extracted ion chromatogram (EIC) illustrating the steady-state signal of a sample mixture comprising six independently TMT-labeled concentrations of an analyte, specifically Leucine Enkephalin. The graph demonstrates the continuous spray provided by a static nanoESI source over a one-minute measurement interval, where the signal intensities of the selected MS/MS transitions remain substantially constant, thereby providing the basis for digital signal accumulation.

**FIG. 2b** is a mobilogram showing the separation of the labeled analyte ions based on their ion mobility. The figure illustrates the separation of precursor *m*/*z* ratios into two distinct conformations based on their Collisional Cross Section (CCS). This gas-phase separation facilitates the purification of the target analyte population from background interferences prior to the fragmentation step.

**FIG. 2c** is a mass spectrum illustrating the detection of six distinct TMT reporter ions released from the fragmented analyte ions. The reporter ions, ranging from *m*/*z* 126.1258 to 131.1425, correspond to different peptide concentrations (Cal1 to Cal5 and a Blank). The relative signal intensities of these reporter ions are used to determine the relative quantity of the analyte across the multiplexed samples.

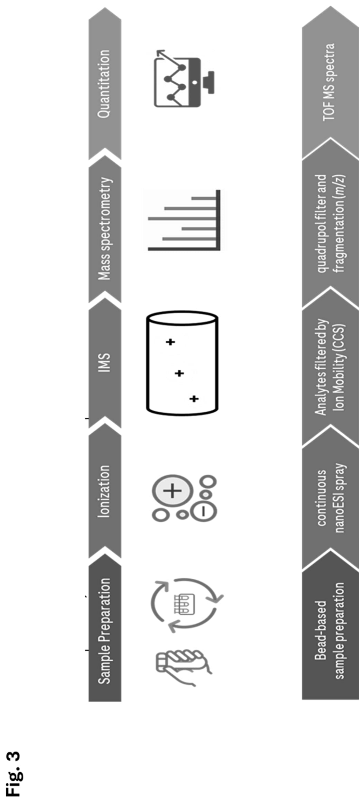

**FIG. 3** is a methodical flow chart for a method and a mass spectrometer system for determining a relative quantity of at least one analyte present in a first biological sample relative to the quantity of the same at least one analyte present in a second biological sample according to an embodiment. The mass spectrometer system may realize a synchronized timing framework which may be used for chromatography-free relative quantification. The system may not only supply the sample via a supply interface but also pre-process the sample by means of a pre-processor for sample preparation.

The pre-processing and/or sample preparation may include: providing the first biological sample comprising the at least one analyte labeled with a first isobaric mass tag; providing the second biological sample comprising the at least one analyte labeled with a second isobaric mass tag different from the first isobaric mass tag; and mixing the first biological sample with the second biological sample to obtain a sample mixture. Alternatively, or in addition, the pre-processing / sample preparation may include a bead-based sample preparation.

A stream of the sample mixture is then introduced by direct inlet into an ionization source where ionizing of the stream of the sample mixture to generate labeled analyte ions of the at least one analyte labeled with the first and labeled with the second isobaric mass tag takes place. The ionization source (denoted also ionizer) may generate a steady-state signal over a defined time span, specifically a continuous nanoESI spray.

The labeled analyte ions are then separated / filtered from other components of the sample mixture based on their ion mobility (CCS), denoted ion mobility separation (IMS).

The separated labeled analyte ions are then fragmented to release reporter ions from the first and second isobaric mass tags. In the present embodiment of **Fig. 3****,** the fragmentation (*m*/*z*) is associated with mass spectrometry using a quadrupole filter.

Signal intensities of the reporter ions at their respective mass-to-charge ratios are then measured by recording TOF MS spectra. The relative quantity of the at least one analyte present in the first biological sample relative to the second biological sample based on the measured signal intensities is then determined.

In a specific embodiment downstream of the ionization source, the ion mobility separator (e.g., a TIMS device) comprises an accumulation section and a separation section. The accumulation section may spatially bunch ions over an accumulation time span, after which they are selectively released into the separation section. A second selectivity enhancer module (e.g., a quadrupole) and a fragmenter module may operate in a pulsed manner over shortened time spans, respectively. A detection module (comprising a detector and a first data processor) may record mass-resolved spectra over recording time spans, which are then summed by a controller over the overall measurement time span.

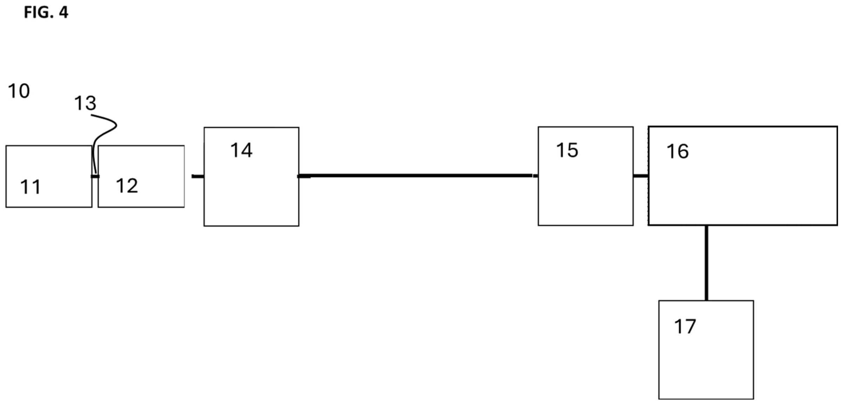

**FIG. 4** is a schematic drawing of a mass spectrometer system 10 according to another embodiment, featuring a multi-stage selectivity architecture for enhanced gas-phase purification. The mass spectrometer system 10 for determining a relative quantity of at least one analyte present in a first biological sample relative to the quantity of the same at least one analyte present in a second biological sample comprises: a sample supply interface 11 configured to introduce a stream of a sample mixture into an ionization source 12 by means of a direct inlet 13, wherein the sample mixture comprises the at least one analyte from the first biological sample labeled with a first isobaric mass tag and the at least one analyte from a second biological sample labeled with a second isobaric mass tag different from the first isobaric mass tag; the ionization source 12 configured to ionize the stream of the sample mixture to generate labeled analyte ions; an ion mobility separator 14 positioned downstream of the ionization source 12 and configured to separate the labeled analyte ions from other components of the sample mixture based on their ion mobility; a fragmenter module 15 configured to fragment the separated labeled analyte ions to release reporter ions from the first and second isobaric mass tags; a detector 16 configured to measure signal intensities of the reporter ions at their respective mass-to-charge ratios; and a controller 17 configured to determine the relative quantity of the at least one analyte present in the first biological sample relative to the second biological sample based on the measured signal intensities.

The detector 16 may comprise a Time-of-Flight (TOF) detector. The controller 17 may be configured to distinguish the reporter ions from the first isobaric mass tag and the second isobaric mass tag based on their differential flight times to the detector, such that the reporter ions are detected sequentially within a single spectral acquisition cycle. The controller 17 may be configured to orchestrate the simultaneous analysis by: a) recording a plurality of time-of-flight transients in which the reporter ions of the first and second isobaric mass tags arrive at the TOF detector at distinct times based on their differential flight times; b) digitally accumulating said transients into a final mass-resolved spectrum; and c) calculating the ratio of the signal intensities of the reporter ions extracted from said final mass-resolved spectrum.

**FIG. 5** is a correlation plot showing the relationship between the concentration of a model peptide (Leucine Enkephalin) and the measured relative signal intensities of the reporter ions (ranging from *m*/*z* 126.1258 to 131.1425). The plot displays a calibration series comprising a blank (0 µM) and five calibration points (Cal1: 90 µM, Cal2: 180 µM, Cal3: 360 µM, Cal4: 900 µM, Cal5: 1800 µM), which were labeled with specific isobaric mass tags TMT-131, TMT-130, TMT-129, TMT-128, TMT-127, and TMT-126, respectively. The signal intensities of the reporter ions exhibit a high degree of linearity across the entire concentration range from 90 µM to 1800 µM. This demonstrates that the method according to the present invention allows for a precise quantitative readout.

### Reference numerals

- 10: Mass spectrometer system
- 11: Sample supply interface
- 12: Ionization source
- 13: Direct inlet
- 14: Ion mobility separator
- 15: Fragmenter module
- 16: Detector
- 17: Controller
- 100: Mass spectrometric method
- 101: Step of providing the first biological sample
- 102: Step of providing the second biological sample
- 103: Step of mixing samples to obtain a sample mixture
- 104: Step of introducing the sample mixture by direct inlet
- 105: Step of ionizing the stream of the sample mixture
- 106: Step of separating labeled analyte ions based on ion mobility
- 107: Step of fragmenting the separated labeled analyte ions
- 108: Step of measuring signal intensities and determining relative quantity
- Cal1-Cal5: TMT-labeled peptide concentrations (calibration standards)
- Blank: Control/Blank sample

## Claims

1. Mass spectrometric method (100) for determining a relative quantity of at least one analyte present in a first biological sample relative to the quantity of the same at least one analyte present in a second biological sample, the method comprising the steps of:
providing (101) the first biological sample comprising the at least one analyte labeled with a first isobaric mass tag;
providing (102) the second biological sample comprising the at least one analyte labeled with a second isobaric mass tag different from the first isobaric mass tag;
mixing (103) the first biological sample with the second biological sample to obtain a sample mixture;
introducing (104), by direct inlet, a stream of the sample mixture into an ionization source;
ionizing (105) the stream of the sample mixture to generate labeled analyte ions of the at least one analyte labeled with the first and labeled with the second isobaric mass tag;
separating (106) the labeled analyte ions from other components of the sample mixture based on their ion mobility;
fragmenting (107) the separated labeled analyte ions to release reporter ions from the first and second isobaric mass tags; and
measuring (108) signal intensities of the reporter ions at their respective mass-to-charge ratios and determining therefrom the relative quantity of the at least one analyte present in the first biological sample relative to the second biological sample based on the measured signal intensities.

2. The mass spectrometric method (100) of claim 1,
wherein providing the first biological sample corresponds to providing a first reaction mixture that comprises: the first biological sample comprising the at least one analyte labeled with the first isobaric mass tag and at least one residual labeling reagent for labeling the at least one analyte with the first isobaric mass tag; and/or
wherein providing the second biological sample corresponds to providing a second reaction mixture that comprises: the second biological sample comprising the at least one analyte labeled with the second isobaric mass tag and at least one residual labeling reagent for labeling the at least one analyte with the second isobaric mass tag,
specifically, wherein the step of mixing the first biological sample with the second biological sample to obtain a sample mixture is followed by diluting the sample mixture in an acidified organic solvent.

3. The mass spectrometric method (100) of claim 2, wherein the acidified organic solvent comprises at least one of: acetonitrile (ACN); methanol (MeOH); ethanol (EtOH), specifically wherein the acidified organic solvent comprises: 60-98 vol% ACN and 0.02-0.4 vol% FA, with the balance to 100 vol% being water specifically 70-95 vol% ACN and 0.05-0.15 vol% FA, with the balance to 100 vol% being water and more specifically 85-93 vol% ACN and 0.08-0.12 vol% FA with the balance to 100 vol% being water.

4. The mass spectrometric method (100) of any one of the preceding claims, wherein separating the labeled analyte ions based on their ion mobility comprises separating the labeled analyte ions from singly charged background ions and/or isobaric interferences.

5. The mass spectrometric method (100) of any one of the preceding claims, wherein the step of ionizing the stream of the sample mixture is at least partially performed by means of a static nano-electrospray ionization (nanoESI) providing a continuous spray of the sample mixture.

6. The mass spectrometric method (100) of any one of the preceding claims,
wherein the stream of the sample mixture is provided as a constant stream, specifically the constant stream is provided for a duration of at least 10 seconds, specifically of at least 20 seconds and more specifically of at least 30 seconds; and/or
wherein the step of measuring signal intensities comprises recording a plurality of mass-resolved spectra over a measurement time period while the stream is introduced, and accumulating said spectra to obtain a final mass-resolved spectrum, specifically wherein the measurement time period is between 5 seconds and 30 minutes, specifically between 10 second and 20 minutes and more specifically between 15 seconds and 10 minutes.

7. The mass spectrometric method (100) of any one of the preceding claims, wherein the mass spectrometric method (100) comprises a simultaneous analysis of the first and second biological samples in a single spectral scan.

8. The mass spectrometric method (100) of any one of the preceding claims being chromatography-free.

9. The mass spectrometric method (100) of any one of the preceding claims, wherein the fragmenting is performed by collision with gas particles.

10. The mass spectrometric method (100) of any one of the preceding claims, wherein separating the labeled analyte ions based on their ion mobility comprises performing Trapped Ion Mobility Spectrometry (TIMS).

11. The mass spectrometric method (100) of any one of the preceding claims, further comprising a step of filtering the labeled analyte ions based on their mass-to-charge ratio using a quadrupole mass filter prior to fragmenting the separated labeled analyte ions, wherein the quadrupole mass filter is configured to transmit the labeled analyte ions and reject ions having mass-to-charge ratios different from the labeled analyte ions.

12. The mass spectrometric method (100) of any one of the preceding claims,
wherein the first biological sample is a patient sample and the second biological sample is a control sample, and wherein determining the relative quantity comprises comparing the measured signal intensity of the reporter ion from the patient sample to the measured signal intensity of the reporter ion from the control sample; or
wherein the first biological sample is a first patient sample and the second biological sample is a second patient sample of the same patient or a different patient, and wherein determining the relative quantity comprises comparing the measured signal intensity of the reporter ion from the first patient sample to the measured signal intensity of the reporter ion from the second patient sample.

13. The mass spectrometric method (100) of any one of the preceding claims, wherein measuring the signal intensities comprises using a Time-of-Flight (TOF) mass spectrometer.

14. A mass spectrometer system (10) for determining a relative quantity of at least one analyte present in a first biological sample relative to the quantity of the same at least one analyte present in a second biological sample, the system comprising:
a sample supply interface (11) configured to introduce a stream of a sample mixture into an ionization source (12) by means of a direct inlet (13), wherein the sample mixture comprises the at least one analyte from the first biological sample labeled with a first isobaric mass tag and the at least one analyte from a second biological sample labeled with a second isobaric mass tag different from the first isobaric mass tag;
the ionization source (12) configured to ionize the stream of the sample mixture to generate labeled analyte ions;
an ion mobility separator (14) positioned downstream of the ionization source (12) and configured to separate the labeled analyte ions from other components of the sample mixture based on their ion mobility;
a fragmenter module (15) configured to fragment the separated labeled analyte ions to release reporter ions from the first and second isobaric mass tags;
a detector (16) configured to measure signal intensities of the reporter ions at their respective mass-to-charge ratios; and
a controller (17) configured to determine the relative quantity of the at least one analyte present in the first biological sample relative to the second biological sample based on the measured signal intensities.

15. The mass spectrometer system of claim 14,
wherein the detector is a Time-of-Flight (TOF) detector; and/or
wherein the controller is configured to distinguish the reporter ions from the first isobaric mass tag and the second isobaric mass tag based on their differential flight times to the detector, such that the reporter ions are detected sequentially within a single spectral acquisition cycle; and/or
wherein the controller is configured to orchestrate the simultaneous analysis by: a) recording a plurality of time-of-flight transients in which the reporter ions of the first and second isobaric mass tags arrive at the TOF detector at distinct times based on their differential flight times; b) digitally accumulating said transients into a final mass-resolved spectrum; and c) calculating the ratio of the signal intensities of the reporter ions extracted from said final mass-resolved spectrum.
